# EUROPEAN PATENT APPLICATION

(11) **EP 4 722 342 A1**
(43) Date of publication of application: **08.04.2026**
(21) Application number: 23939810.0
(22) Date of filing: 26.05.2023
(51) Int. Cl.: C12N 5/071, C12N 7/00, A61K 39/12, A61P 31/12, A61K 39/00

(54) **CELL LINE FOR CULTURING AFRICAN SWINE FEVER VIRUS AND VACCINE USING SAME**

(71) Applicant: ChoongAng Vaccine Laboratories Co., Ltd (CAVAC), Daejeon 34055 (KR)
(72) Inventor: YOON, In-joong, Daejeon 34055 (KR); LEE, Joo Young, Daejeon 34055 (KR); YOO, Sung-sik, Daejeon 34055 (KR); KWON, Hyeok-Il, Daejeon 34055 (KR); LEE, Seung Chul, Daejeon 34055 (KR); KIM, Min Ho, Daejeon 34055 (KR)
(74) Representative: Ipsilon
(86) International application number: PCT/KR2023/007253
(87) International publication number: WO 2024/248170

(57) **Abstract**

The present invention relates to a CA-CAS-01-A cell line, deposited under Accession Number KCTC14568BP, which is capable of mass propagation of African swine fever virus (ASFV), and to a use thereof. The cell line of the present invention and the ASFV produced therefrom can be advantageously utilized for the prevention of African swine fever and the mitigation of its impact.

## Description

### TECHNICAL FIELD

The present invention relates to a cell line for culturing African swine fever virus and a vaccine using the same.

This study was supported by the program for developing technology to cope with emerging diseases of the Ministry of Agriculture, Food and Rural Affairs, South Korea (Project No. 119081053SB010).

### BACKGROUND ART

African swine fever (ASF) is a viral hemorrhagic disease that affects only animals belonging to the family *Suidae.* It is primarily transmitted through direct contact with secretions or excretions from infected pigs. The African swine fever virus (ASFV), the causative agent of the disease, is classified according to its pathogenicity into highly virulent, moderately virulent, and low virulent strains. Highly virulent strains typically cause peracute (death of pigs within 1-4 days after the infection) or acute (death within 3-8 days after the infection) forms of the disease. Moderately virulent strains cause acute (death within 11-15 days) or subacute (death around 20 days after the infection) forms of the disease. Low virulent strains have been reported only in regions where the disease has become endemic and cause subclinical or chronic forms of the disease. The mortality rate is nearly 100% in cases caused by highly virulent viruses, whereas in chronic forms it is reported to be below 20%. ASFV belongs to the family *Asfarviridae* and is a relatively large virus with a double-stranded DNA genome of about 189,000 base pairs containing more than 189 genes and an icosahedral structure. It is known to have 23 genotypes.

Various studies have been conducted to develop vaccines for the prevention or treatment of African swine fever. The studies include various researches carried out by many research groups using blood leukocytes, purified and inactivated virions, glutaraldehyde-fixed macrophages infected with the virus, culture supernatants of detergent-treated infected alveolar macrophages, DNA vaccines, attenuated live virus vaccines, inactivated vaccines, and viral vector-based vaccines. However, ASFV exhibits high genetic diversity and complex structural characteristics, and it is difficult to infect common cell types. Therefore, culturing or isolating the virus for vaccine production using PAM (porcine alveolar macrophage) primary cells, known to be susceptible to virus, remains extremely challenging. Although ASFV Georgia strains have been reported to adapt and proliferate in Vero cell lines, their protective efficacy as vaccines is known to be significantly low. Similarly, Spanish ASFV strains have been found unsuitable for vaccine production. In general, ASFV shows limited improvement in viral propagation property even after multiple serial passages. While ASFV strains adapted to Vero cell lines for more than 100 passages exhibit good stability and propagation property in cells, it is found that they do not demonstrate effective protective immunity when used as vaccines.

Meanwhile, Korean Patent Publication No. 2011-0123918 discloses "PAM-pCD163 cell line for isolation of porcine reproductive and respiratory syndrome virus and vaccine using the same," and Korean Patent Publication No. 2015-0019910 discloses "Immortalized porcine kidney-derived cell line and method for propagating porcine circovirus type 2 using the same." However, there is no disclosure regarding the "Cell line for culturing African swine fever virus and vaccine using the same" described in the present invention.

### DETAILED DESCRIPTION

### TECHNICAL PROBLEMS TO BE SOLVED

The present invention is devised in view of the aforementioned needs. In order to establish a cell line capable of propagating the African swine fever virus (ASFV), the inventors of the present invention infected various animal-derived kidney cell lines with ASFV and continuously performed serial passages while examining whether ASFV could be isolated and propagated. As a result, it was found that the MA104 cell line (African green monkey kidney cell) is capable of continuously propagating ASFV for more than 10 passages, unlike other kidney cell lines. Furthermore, among the single-cell clones derived from the MA104 cell line, a particular single-cell clone was identified that exhibited superior ASFV propagation ability compared to other clones and had the characteristic of producing a naturally attenuated ASFV. This single-cell clone was designated as the CA-CAS-01-A cell line, thereby completing the present invention.

### TECHNICAL MEANS FOR SOLVING PROBLEMS

To solve the problems that are descried above, the present invention provides CA-CAS-01-A cell line, deposited under Accession Number KCTC14568BP, which is capable of mass production of African swine fever virus.

The present invention further provides a method for mass production of African swine fever virus, comprising infecting the above-mentioned cell line with ASFV and subsequently culturing it.

The present invention still further provides a vaccine composition comprising African swine fever virus produced by the aforementioned method.

### ADVANTAGEOUS EFFECT OF THE INVENTION

The CA-CAS-01-A cell line of the present invention is a novel cell line for the propagation of African swine fever virus (ASFV), which enables the isolation and cultivation of ASFV. The ASFV produced (i.e., propagated) from this cell line of the present invention is in naturally attenuated and non-virulent form, and thus can be effectively utilized for the prevention of African swine fever and the mitigation of its impact.

### BRIEF DESCRIPTION OF DRAWINGS

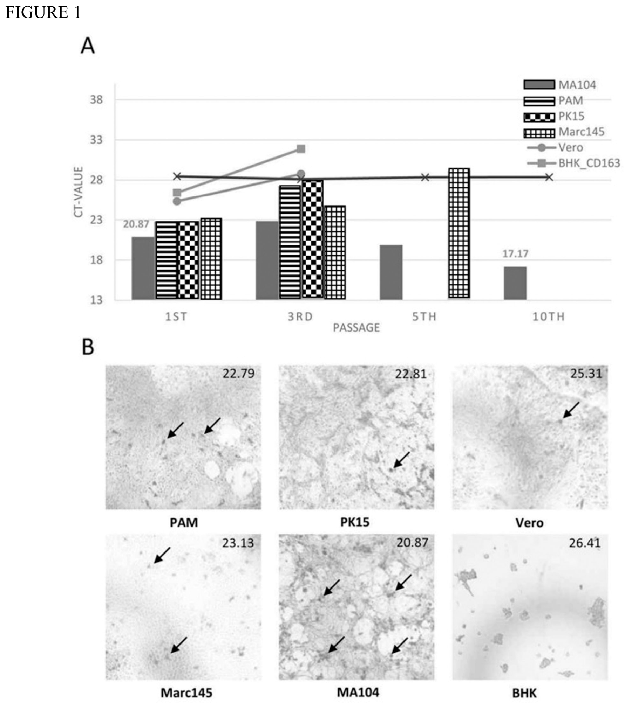

Figure 1 shows the results of determining susceptibility of various cell lines to African swine fever virus (ASFV). Panel A presents the ASFV gene expression levels in each cell line according to passages. Panel B shows cell images of each cell line at passage 1. The numerical values in the upper right corner of the images in B indicate the Ct values obtained by measuring the ASFV P72 gene in real time, representing the susceptibility of each cell line to ASFV. Arrows indicate regions within the cytoplasm where ASFV infection was found by detection of the ASFV P30 gene.

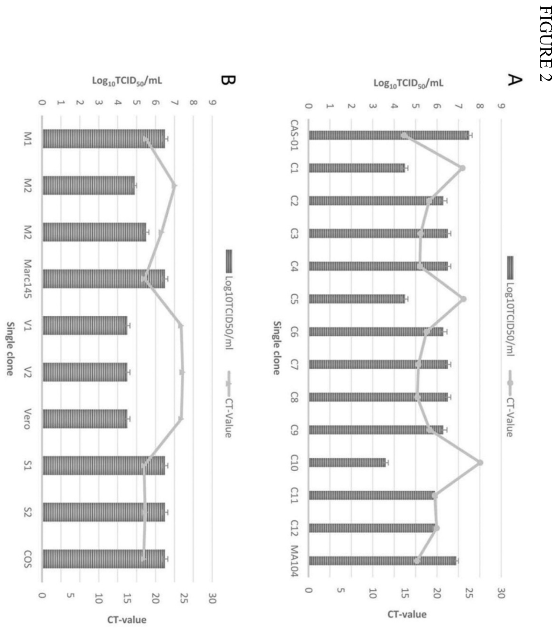

Figure 2A shows the susceptibility of parental MA104 cells and MA104-derived single-cell clones to ASFV, analyzed at passage 7 based on Ct values and TCID₅₀. Figure 2B shows the results of susceptibility analysis for Marc145, Vero, and COS cells and their respective single-cell clones to ASFV. CAS-01 (=CA-CAS-01-A) and C1-C12: MA104 single-cell clones; M1-M3: Marc145 single-cell clones; V1 and V2: Vero single-cell clones; S1 and S2: COS single-cell clones.

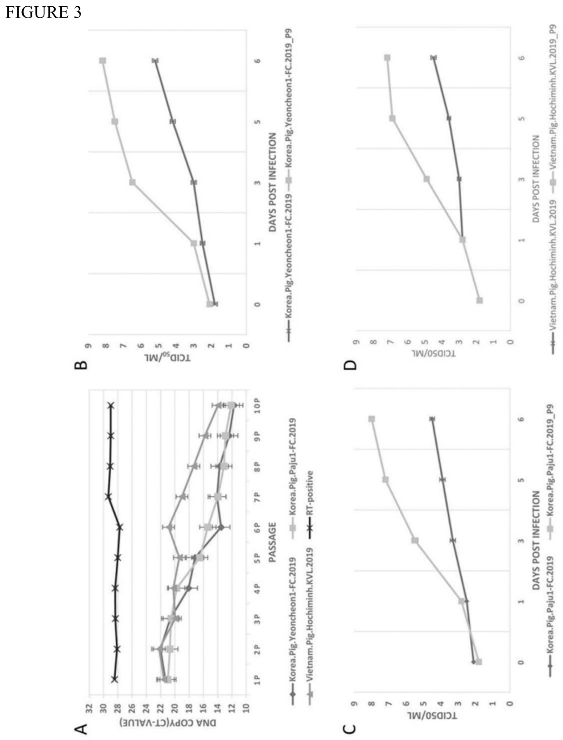

Figure 3 shows the TCID₅₀ results for analyzing the viral titer according to passages in the CA-CAS-01-A cell line infected with an ASFV isolate.

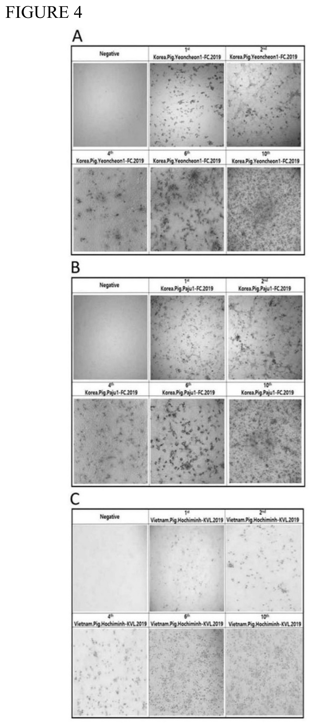

Figure 4 presents cell images showing the cell adaptation process in the CA-CAS-01-A cell line at passages 1, 2, 4, 6, and 10 after serial passaging with two Korean isolates (Korea.Pig.Yeoncheon1-FC.2019 and Korea.Pig.Paju1-FC.2019) or one Vietnamese isolate (Vietnam.Pig.Hochiminh-KVL.2019), determined by using the ASFV P30 gene.

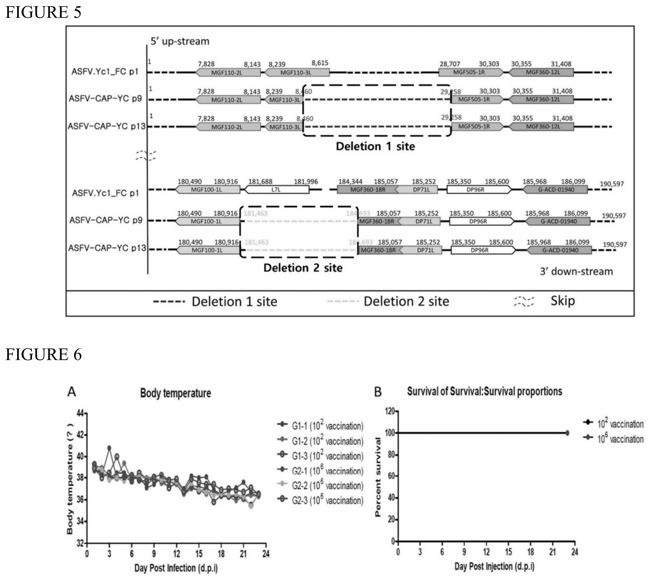

Figure 5 is a schematic diagram showing the genomic deletion sites of ASFV produced in the CA-CAS-01-A cell line infected with the ASFV isolate Korea.Pig.Yeoncheon1-FC.2019. ASFV.Yc1_FC p1: ASFV recovered after one passage; ASFV-CAP-YC p9: ASFV recovered after nine passages; ASFV-CAP-YC p13: ASFV recovered after thirteen passages.
Figure 6 shows the analysis results of body temperature (A) and survival rate (B) in pigs inoculated with ASFV produced in the CA-CAS-01-A cell line.

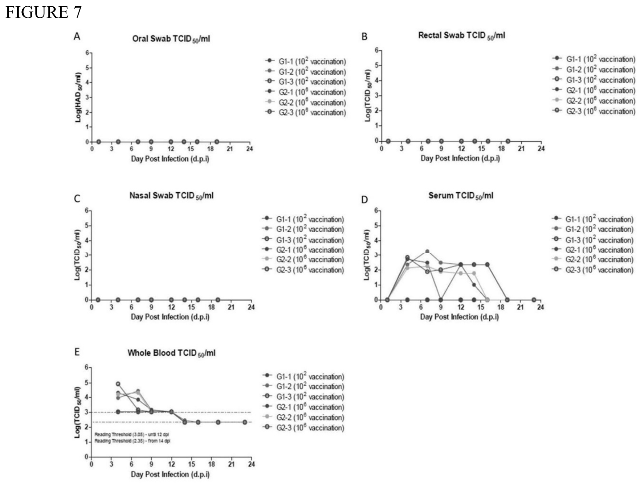

Figure 7 shows the results of viral titer analysis in oral swabs (A), rectal swabs (B), nasal swabs (C), serum (D), and whole blood (E) collected from pigs inoculated with ASFV produced in the CA-CAS-01-A cell line.

### BEST MODE(S) FOR CARRYING OUT THE INVENTION

To achieve the objective of the present invention, the present invention provides CA-CAS-01-A cell line, deposited under Accession Number KCTC14568BP, which is capable of mass propagation of African swine fever virus (ASFV).

The CA-CAS-01-A cell line according to the present invention is derived from the MA104 cell line (African green monkey kidney cell line), and it was established from a cell clone capable of allowing ASFV propagation even after continuous serial passages (up to passage 16), by infecting MA104 cells with an ASFV isolate (field strain), performing serial passaging, and observing viral propagation. In addition, although the CA-CAS-01-A cell line was infected with the ASFV field strain (Korea.Pig.Yeoncheon1-FC.2019), the resulting virus, designated ASFV-CAP-YC, is characterized by being naturally attenuated. Accordingly, the inventors of the present invention deposited the CA-CAS-01-A cell line with Korea Collection for Type Cultures (KCTC) of the Korea Research Institute of Bioscience and Biotechnology (KRIBB) on May 12, 2021, under Accession Number KCTC14568BP.

The term "attenuation" refers to a process in which a gene involved in the essential metabolism of a pathogen undergoes mutation, rendering the pathogen incapable of causing disease in the host while stimulating the immune system to induce immunity. Viral attenuation can be achieved by ultraviolet (UV) irradiation, chemical treatment, or high-level serial passaging *in vitro.* Attenuation can also be accomplished by introducing defined genetic changes, for example, through specific deletions of viral sequences known to confer virulence or by inserting such sequences into the viral genome.

The present invention further provides a method for mass production of African swine fever virus, the method comprising:
(a) infecting the CA-CAS-01-A cell line with an African swine fever virus (ASFV) isolate followed by culturing; and
(b) purifying African swine fever virus from a culture medium obtained in the step (a).

In the method according to one embodiment of the present invention, the ASFV isolate used in the step (a) is preferably Korea.Pig.Yeoncheon1-FC.2019, Korea.Pig.Paju1-FC.2019, or Vietnam.Pig.Hochiminh.KVL.2019, but it is not limited thereto.

In addition, the infection may be carried out by treating the CA-CAS-01-A cell line with an ASFV-containing culture medium or a homogenate of animal tissue infected with ASFV, but it is not limited thereto. The time required for infection and the virus propagation period may vary depending on the type of virus; generally, the infection period is about 3 to 12 hours, and the propagation period is about 3 to 7 days.

In addition, in the method according to the present invention, the ASFV obtained in the step (b) may be a naturally attenuated virus, but it is not limited thereto.

In addition, the culture of the CA-CAS-01-A cell line may be carried out using conventional methods and media that are generally known in the pertinent art.

In the method according to the present invention, when infection and propagation of ASFV are completed, the CA-CAS-01-A cells and the culture supernatant are collected and the ASFV can be recovered through purification by conventional methods.

The present invention still further provides a vaccine composition comprising African swine fever virus produced by the aforementioned method.

The vaccine composition according to the present invention can be prepared using the propagated viral culture medium obtained from the culture step of the CA-CAS-01-A cell line infected with ASFV, and may be formulated as a live vaccine or an inactivated vaccine. In the case of an inactivated vaccine, the virus may be inactivated by a specific method such as treatment with formalin or binary ethylenimine (BEI), followed by purification of the inactivated virus using conventional methods. The purified virus stock solution is then subjected to sterility and viral content tests, and the vaccine is prepared by dilution and mixing with preservatives and buffered saline or by adding stabilizers.

Since the ASFV produced using the CA-CAS-01-A cell line according to the present invention is a naturally attenuated virus, the vaccine composition is preferably in the form of an attenuated live vaccine, but it is not limited thereto.

An attenuated vaccine is a live vaccine in which the virulence of a pathogen has been reduced while the organism remains alive. In the case of an attenuated live vaccine, both antibody-mediated humoral response and cellular immune responses can be induced, and since the immunogenicity is sustained, it is advantageous in that it rarely requires additional injections of adjuvants to enhance the efficacy.

In the vaccine composition according to one embodiment of the present invention, the naturally attenuated ASFV produced using the CA-CAS-01-A cell line may have deletion of one or more genes selected from the group consisting of *MGF 110-3L partial, MGF 110-4L, MGF 1105L6L, MGF 110-7L, 285L, ASFV G ACD 00160, MGF 100-1R, ASFV G ACD 00190, MGF 110-9L, MGF 110-11L*_*14L, ASFV G ACD 00240, MGF 110-12L, MGF 110-13La*_*b, ASFV G ACD 00270, MGF 360-4L, MGF 360-6L, MGF 300-1L, MGF 300-2R, MGF 300-4L, MGF 360-8L, MGF 360-9L, MGF 360-10L, MGF 360-11L, MGF 505-1R, L7L, L8L, L9R, L10L,* and *L11*. More preferably, the ASFV may be attenuated by deletion of all of the following genes: *MGF 110-3L partial, MGF 110-4L, MGF 1105L6L, MGF 110-7L, 285L, ASFV G ACD 00160, MGF 100-1R, ASFV G ACD 00190, MGF 110-9L, MGF 110-11L_14L, ASFV G ACD 00240, MGF 110-12L, MGF 110-13La*_*b, ASFV G ACD 00270, MGF 360-4L, MGF 360-6L, MGF 300-1L, MGF 300-2R, MGF 300-4L, MGF 360-8L, MGF 360-9L, MGF 360-10L, MGF 360-11L, MGF 505-1R, L7L, L8L, L9R, L10L,* and *L11*, but it is not limited thereto.

The naturally attenuated ASFV of the present invention may have deletions corresponding to nucleotides 8,460 to 29,258 (Deletion site 1) and nucleotides 181,463 to 184,693 (Deletion site 2) based on the sequence of the African swine fever virus isolate ASFV Georgia 2007/1 genome assembly, complete genome (NCBI Reference Sequence: NC_044959.2). The Deletion sites 1 and 2 contain multiple genes associated with the attenuation that is described above.

In addition, the vaccine composition of the present invention may further comprise a pharmaceutically acceptable carrier and/or adjuvant. Furthermore, in addition to the carrier, the vaccine composition of the present invention may additionally comprise an excipient and/or diluent.

The term "pharmaceutically acceptable," as used herein, refers to substances that are physiologically acceptable and do not cause adverse responses such as gastrointestinal disturbances, dizziness, or similar allergic responses when administered to humans. Examples of such carriers, excipients, and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil. The composition may also further comprise fillers, anti-caking agents, lubricants, wetting agents, flavoring agents, emulsifiers, and preservatives.

The term "adjuvant" refers to a pharmaceutical or immunological substance administered for the purpose of enhancing the immune response caused by a vaccine. Examples of the suitable adjuvants that can be used include aluminum hydroxide, aluminum phosphate, alum (potassium aluminum sulfate), MF59, virosome, AS04 [a mixture of aluminum hydroxide and monophosphoryl lipid A (MPL)], AS03 (a mixture of DL-α-tocopherol, squalene, and the emulsifier polysorbate 80), CpG, flagellin, poly I:C, AS01, AS02, ISCOMs, or ISCOMATRIX.

The vaccine composition of the present invention can be formulated using methods that are well known in the pertinent art to allow rapid release, sustained release, or delayed release of the active ingredient upon administration to a mammal. The formulation may include forms such as powder, granule, tablet, emulsion, syrup, aerosol, soft or hard gelatin capsule, sterile injectable solution, or sterile powder.

The vaccine composition according to the present invention can be administered via various routes, including oral, parenteral (e.g., suppository, transdermal, intravenous, intraperitoneal, intramuscular, intralesional, intranasal, or intrathecal) administration, and may also be administered using implant devices designed for sustained, continuous, or repeated release. The frequency of administration may vary within a desired range, such as once or several times a day, and the duration of administration is also not particularly limited.

Hereinafter, the present invention will be described in greater detail with reference to the following Examples. However, the following Examples are provided solely to exemplify the present invention, and it is evident that the scope of the present invention is not limited to the Examples.

### Examples

### Example 1. Screening of susceptible cell lines for ASFV isolate infection

The inventors of the present invention screened ASFV- susceptible cell lines for ASFV cultivation. The cell lines used were MA104, PAM, PK15, Marc145, Vero, and BHK CD163, all provided by ChoongAng Vaccine Laboratories Co., Ltd. For cell maintenance, α-MEM medium supplemented with 5% FBS and 1% antibiotics was used for all cell lines, and the cells were maintained by subculturing them every 2 to 3 days under conditions of 37°C and 5% CO₂.

**[Table 1]**

| Cells lines used in the present invention | |
|---|---|
| Name of cell line | Origin |
| MA104 | African green monkey kidney |
| PAM | Porcine macrophages |
| PK15 | Porcine kidney |
| Marc 145 | Clone of African green monkey kidney cell line MA-104 |
| Vero | African green monkey kidney epithelia |
| BHK CD163 | Baby hamster kidney |
| COS | African green monkey kidney fibroblast-like cell line |

To determine the susceptibility of each cell line to ASFV, cells were seeded into a 75 cm² flask at a density of 1 × 10⁶ cells per well, and ASFV culture medium (Animal and Plant Quarantine Agency: approximately 18 Ct value; Vietnam Raho-6: approximately 18 Ct value) was applied to infect the cells. Subsequently, the cells were subjected to serial passage at 7-day intervals under conditions of 37°C and 5% CO₂. The culture media were collected at the 1st, 3rd, 5th, and 10th passages, and both the supernatant and cells were frozen at -80°C and thawed once in a 37°C warm water bath. The resulting culture medium was then used for qPCR (quantitative PCR) analysis. During the serial passage, cells were infected using the culture medium from the previous passage. qPCR was performed under the following conditions using a primer set specific for the ASFV P72 gene. The positive control used was the domestic isolate Korea.Pig.Paju1-FC.2019.

**[Table 2]**

| Information of primers used for qPCR | | |
|---|---|---|
| Primer name | Nucleotide sequence (5'→3') | SEQ ID NO: |
| P72_Forward | CTG CTC ATG GTA TCA ATC TTA TCG A | 1 |
| P72_Reverse | GAT ACC ACA AGA TC(AG) GCC GT | 2 |

**[Table 3]**

| Conditions for qPCR | | |
|---|---|---|
| Step | Reaction conditions | Number of step |
| UNG (Uracil-DNA Glycosylase) treatment | 50°C, 2 minutes | 1 |
| Pre-denaturation | 95°C, 10 minutes | 1 |
| Denaturation | 95°C, 15 seconds | 40 |
| Annealing | 58°C, 1 minute | |

As a result, it was found that only the MA104 cell line continuously supported ASFV propagation even at the 10th passage (Figure 1). Therefore, the MA104 cell line was used to establish a cell line for ASFV propagation.

### Example 2. Comparison of ASFV-specific susceptibility among single-cell clones derived from MA104 cell line

The inventors of the present invention diluted the MA104 cell line stepwise to obtain single-cell clones. After infecting those clones with ASFV under the same conditions as in Example 1, a total of seven serial passages were performed. qPCR was then conducted using a primer set specific to the ASFV P72 gene, and viral titers were quantified using the TCID₅₀ (tissue culture infective dose 50) assay. In addition, CAS-01 and the Marc145 and Vero cell lines, both having the same parental cells (MA104), as well as their single-cell clones, and the COS cell line derived from African green monkey kidney and its single-cell clone were used for the infection and compared to one another under the same conditions.

As a result, the MA104 cell line showed a viral titer of 6.9 log₁₀TCID₅₀/mL, while the cell clone designated as CAS-01 showed a viral titer of 7.5 log₁₀TCID₅₀/mL. In contrast, another single-cell clone derived from MA104, designated as C10, showed a viral titer of 3.6 log₁₀TCID₅₀/mL, indicating a significant difference in viral propagation property among the single-cell clones (Figure 2A). The Marc145 and Vero cell lines showed viral titers of 6.5 log₁₀TCID₅₀/mL and 4.5 log₁₀TCID₅₀/mL, respectively, while the COS cell line showed a viral titer of 6.5 log₁₀TCID₅₀/mL (Figure 2B). These results demonstrate that even the cell lines originating from the same origin exhibit differences in viral susceptibility depending on the single-cell clone. Therefore, the CAS-01 cell line was selected for further experiments to support ASFV replication and propagation. This cell line was designated as "CA-CAS-01-A" and was deposited with Korea Collection for Type Cultures (KCTC) of the Korea Research Institute of Bioscience and Biotechnology (KRIBB) on May 12, 2021, under Accession Number KCTC 14568BP.

### Example 3. Viral propagation property of ASFV in CA-CAS-01-A cell line according to passage after viral infection

The inventors of the present invention analyzed viral propagation property in the CA-CAS-01-A cell line following serial passages after infection with ASFV isolates Korea.Pig.Yeoncheon1-FC.2019, Korea.Pig.Paju1-FC.2019, or Vietnam.Pig.Hochiminh.KVL.2019. Each virus isolate was provided by Dr. Yong-Joo Kim from the Animal and Plant Quarantine Agency, South Korea. The infection concentration of the virus was the same as described in Example 1.

As a result, when the CA-CAS-01-A cell line was infected with each initial ASFV isolate (passage 1), followed by cultivation, the viral titers were measured as 5.2 log₁₀TCID₅₀/mL for the Korea.Pig.Yeoncheon1-FC.2019 isolate, and 4.5 log₁₀TCID₅₀/mL for both the Korea.Pig.Paju1-FC.2019 and Vietnam.Pig.Hochiminh.KVL.2019 isolates. Viral propagation property was determined according to days after the infection, in which the determination has been made with each ASFV isolate obtained from a total of nine serial passages. As a result, on day 6 after the infection, the viral titers were 8.2 log₁₀TCID₅₀/mL for the Korea.Pig.Yeoncheon1-FC.2019 isolate, 8.0 log₁₀TCID₅₀/mL for the Korea.Pig.Paju1-FC.2019 isolate, and 7.2 log₁₀TCID₅₀/mL for the Vietnam.Pig.Hochiminh.KVL.2019 isolate. These results indicate that all three ASFV isolates exhibited significantly enhanced propagation property after serial adaptation passages in the CA-CAS-01-A cell line (Figures 3 and 4).

### Example 4. Characteristics of ASFV propagated in CA-CAS-01-A cell line

To examine the characteristics of ASFV propagated in the CA-CAS-01-A cell line, the inventors of the present invention recovered viruses from culture media after 1, 9, and 13 serial passages and analyzed their genome sequences using the MiSeq System (Illumina, USA) and the CLC Genomics Workbench program. As a result, as shown in Figure 5, ASFV recovered after the 9th and 13th passages (designated ASFV-CAP-YC p9 and ASFV-CAP-YC p13, respectively) exhibited large genomic deletions in both the 5' and 3' regions compared to the genome of the initial infected virus. The genes located within each deletion site are listed in Table 4 below. Notably, many of the deleted genes were identified as being associated with the attenuation.

**[Table 4]**

| Deleted sites in ASFV (ASFV-CAP-YC) produced from CA-CAS-01-A cell line | |
|---|---|
| | Deleted Genes |
| Deletion site 1 | **MGF 110-3L partial, MGF 110-4L, MGF 1105L6L, MGF 110-7L, 285L, ASFV G ACD 00160, MGF 100-1R, ASFV G ACD 00190. MGF 110-9L, MGF 110-11L 14L, ASFV G ACD 00240 MGF 110-12L, MGF 110-13La b, ASFV G ACD 00270, MGF 360-4L, MGF 360-6L, MGF 300-1L, MGF 300-2R, MGF 300-4L, MGF 360-8L, MGF 360-9L, MGF 360-10L, MGF 360-11L, MGF 505-1R.** ASF G ACD 00120, MGF 110-8L, ASF G ACD 00210, ASF G ACD 00300, ASF G ACD 00320, ASF G ACD 00330, ASF G ACD 00350, ASF G |
| | ACD 00360, X69R |
| Deletion site 2 | **L7L. L8L, L9R, L10L, L11L,** ASF G ACD 01870, MGF 100-3L, MGF 360-18R |
| **Bold:** Genes affecting the attenuation | |

### Example 5. Determination of attenuation (safety) of ASFV virus antigen (ASFV-CAP-YC) propagated in CA-CAS-01-A cell line

To verify the attenuation (safety) of the ASF antigen (ASFV) propagated and attenuated through 14 serial passages in the CA-CAS-01-A cell line, the inventors of the present invention inoculated 3-week-old male pigs (Landrace or Danish Landrace) with ASFV at concentrations of 10² or 10⁶ TCID₅₀/mL. As a result, as shown in Figure 6, no changes in body temperature or survival rate were observed in any of the pigs up to 24 days post-injection. Furthermore, as shown in Figure 7, no virus was detected in oral, rectal, or nasal swab samples. In serum, the virus was detected at levels of 10²-10³ TCID₅₀/mL on day 3 post-injection of the antigen, but it completely disappeared by day 19. In whole blood, the virus was detected at levels of 10³-10⁵ TCID₅₀/mL, which gradually decreased over time. Additionally, all inoculated pigs were monitored for three weeks for body temperature, survival rate, and clinical symptoms. As a result, all groups showed 100% survival rate, and no clinical symptoms specific to ASFV infection were observed.

### [Accession Information]

Depositary Authority: Korea Research Institute of Bioscience and Biotechnology (KRIBB)
Accession Number: KCTC14568BP
Date of Deposit: May 12, 2021

## Claims

1. CA-CAS-01-A cell line, deposited under Accession Number KCTC14568BP, which is capable of propagating African swine fever virus.

2. The CA-CAS-01-A cell line according to Claim 1, wherein the CA-CAS-01-A cell line is derived from MA104 cell line (African green monkey kidney cell line).

3. The CA-CAS-01-A cell line according to Claim 1, wherein the CA-CAS-01-A cell line produces attenuated African swine fever virus during serial passage culture after infection with an African swine fever virus isolate.

4. A method for mass production of African swine fever virus, the method comprising:
(a) infecting the CA-CAS-01-A cell line of Claim 1 with an African swine fever virus isolate followed by culturing; and
(b) purifying African swine fever virus from a culture medium obtained in the step (a).

5. The method according to Claim 4, wherein the African swine fever virus of the step (b) is an attenuated virus.

6. A vaccine composition for prevention of African swine fever comprising African swine fever virus produced by the method of Claim 4.

7. The vaccine composition according to Claim 6, wherein the African swine fever virus is a naturally attenuated virus.

8. The vaccine composition according to Claim 7, wherein the attenuation represents deletion of one or more genes selected from the group consisting of *MGF 110-3L partial, MGF 110-4L, MGF 1105L6L, MGF 110-7L, 285L, ASFV G ACD 00160, MGF 100-1R, ASFV G ACD 00190, MGF 110-9L, MGF 110-11L*_*14L, ASFV G ACD 00240 MGF 110-12L, MGF 110-13La*_*b, ASFV G ACD 00270, MGF 360-4L, MGF 360-6L, MGF 300-1L, MGF 300-2R, MGF 300-4L, MGF 360-8L, MGF 360-9L, MGF 360-10L, MGF 360-11L, MGF 505-1R, L7L, L8L, L9R, L10L* and L11.

9. The vaccine composition according to Claim 6, wherein the composition further comprises a pharmaceutically acceptable carrier or adjuvant.
